# EUROPEAN PATENT SPECIFICATION

(11) **EP 0 666 735 B1**
(45) Date of publication and mention of the grant of the patent: **07.10.1998**
(21) Application number: 93924575.9
(22) Date of filing: 02.11.1993
(51) Int. Cl.: A61K 7/48, A61K 7/42

(54) **RETINOL CONTAINING COSMETIC COMPOSITION**
RETINOL ENTHALTENDE KOSMETISCHE ZUSAMMENSETZUNG
COMPOSITIONS COSMETIQUES CONTENANT DU RETINOL

(30) Priority: 05.11.1992 GB 9223235
(43) Date of publication of application: 16.08.1995
(73) Proprietor: UNILEVER PLC, London EC4P 4BQ (GB); UNILEVER N.V., 3013 AL Rotterdam (NL)
(72) Inventor: HARDING, Clive Roderick, Rushden, Northampton NN10 9YB (GB); LEE, Caroline Marian, Ampthill, Bedford MK45 2LE (GB); SCOTT, Ian, Richard, Allendale, NJ 07401 (US)
(74) Representative: Evans, Jacqueline Gail Victoria
(86) International application number: EP9303064
(87) International publication number: WO9409756

(56) References cited:
- EP-A- 0 470 275
- EP-A- 0 512 814
- WO-A-93/00085
- DATABASE WPI Week 9334, Derwent Publications Ltd., London, GB; AN 93269739 & JP,5 186 324 (SUNSTAR CHEM IND) 27 July 1993
- DATABASE WPI Week 9208, Derwent Publications Ltd., London, GB; AN 92062027 & JP,A,4 009 325 (SUNSTAR KK) 14 January 1992
- PATENT ABSTRACTS OF JAPAN vol. 13, no. 472 (C-647)25 January 1989 & JP,A,01 186 811 (SUNSTAR INC) 26 July 1989

## Description

### FIELD OF INVENTION

The invention relates to the use of a composition for topical application to human skin in order to lighten skin colour and to promote the repair of photo-damaged skin and/or to reduce or prevent the damaging effects of ultra-violet light on skin.

### BACKGROUND TO THE INVENTION

The treatment of human skin damaged due to exposure to ultra-violet light, ie. photo-damage, has been subject to much research effort in recent years, particularly with the realisation that skin cancer and other skin disorders can arise where the exposure to sunlight is excessive. This problem is even more serious with the depletion of the ozone layer which is believed to permit a higher level of ultra-violet radiation to reach the earth's surface.

Chronic exposure to sunlight results in multiple adverse effects on all structural elements of the skin. The clinical manifestation of these changes, collectively known as photoageing is lax, dry inelastic skin that is wrinkled and blotchy with a coarse, roughened texture, uneven colour and altered pigmentation.

Skin blotchiness or mottling (hyperpigmentation) is due to changes in the melanocytes or the processing of melanin within the population of epidermal cells. These pigment producing cells, which unlike the keratinocytes remain at the base of the epidermis, lose their normal regulation process with ageing and produce excess pigment. This leads to the formation of dense perinuclear clumps of melanin in slowly turning over keratinocytes within the epidermis, and areas of hyperpigmentation or 'age spots' develop.

In the therapy of such hyperpigmented skin, certain skin lightening agents such as kojic acid, hydroquinone or ascorbic acid are effective by inhibiting the formation of melanin. Vitamin A acid (retinoic acid) is beneficial in hyperpigmentation problems by normalising the skin colour.

Also by increasing cell turnover, Vitamin A acid prevents accumulation of pigment within the more rapidly dividing and migrating keratinocytes. Vitamin A acid also enhances the pigment reducing potential of conventional skin lightening agents.

The topical application of Vitamin A acid does however have a major drawback in that it is a potent skin irritant, and can accordingly damage the skin. Vitamin A acid is also a teratogen. Its recommended use for example as a prescription drug in the treatment of acne involves careful control, such that excessive doses are avoided in order to restrict the side effects which can occur with skin. By the same token, the use of Vitamin A acid for lightening skin and in the treatment or prevention of photo-damaged skin is severely limited by these side effects, and for this reason it is not generally recommended for use in cosmetic products.

Derwent abstract 92-062027 relating to J04009325 describes material for skin lightening which comprises Mannentake mushroom mycelium cultured substance and/or its extract and at least one of ascorbic acid, its salt or derivative, retinol, its salt or derivative, pyridoxine, its salt or derivative, panthothenic acid, its salt or derivative and tocopherol, its salt or derivative.

WO 93/00085, published after the priority date of the present invention, discloses skin care compositions comprising a water-in-oil emulsion base containing retinoids and antioxidants and possibly chelating agents. The antioxidants and/or chelating agents serve to prevent breakdown of the retinoid and thereby obtaining good keepability of the product. Amongst the many possible antioxidants that can be used, e.g. ascorbic acid and hydroquinone are mentioned.

EP 512 814 was also published after the priority date of the present invention. It concerns topical compositions for preventing or at least reducing the damaging effects of ultra-violet light on skin that comprise 1-hydroxy cholecalciferol and/or 1,25 dihydroxycholecalciferol in combination with a sunscreen material and preferably also retinol and/or a derivative thereof. The composition may further comprise a wide range of optional components, e.g. skin lightening agent.

We have now discovered that retinol or certain derivatives thereof, when combined with a skin lightening agent can be used effectively in the repair of photo-damaged skin or the prevention of photo-damage to skin following exposure to ultra-violet light. This combination is also particularly useful in reducing hyperpigmentation of skin.

The invention is accordingly concerned with the use of retinol or certain derivatives thereof together with a skin lightening agent in compositions for topical application to human skin, to lighten skin and for treatment of photodamaged and/or hyperpigmented skin or the prevention thereof, following excessive exposure to ultra-violet light

### DEFINITION OF THE INVENTION

Accordingly, the invention provides the use of an effective amount of from 0.01 to 20% by weight of a skin lightening agent chosen from L-ascorbic acid and derivatives thereof, kojic acid and derivatives thereof, hydroquinone and derivatives thereof, extract of placenta, arbutin, niacin, niacinamide, α hydroxy acids, phloretin, phloridzin, liquorice extract, cysteaminylphenol and derivatives thereof and compounds having the structure (2): 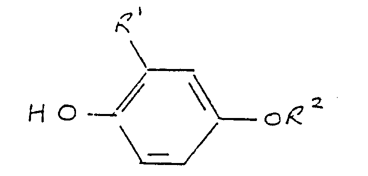
where R¹ represents H, or an ether group represented by OR³,
R² and R³ are the same or different and each represents a group chosen from branched or unbranched alkyl or alkenyl groups having an average of from 1 to 20 carbon atoms,
in a cosmetic composition which composition comprises an effective amount of from 0.001 to 10% by weight of retinol or a derivative thereof having the structure (1): 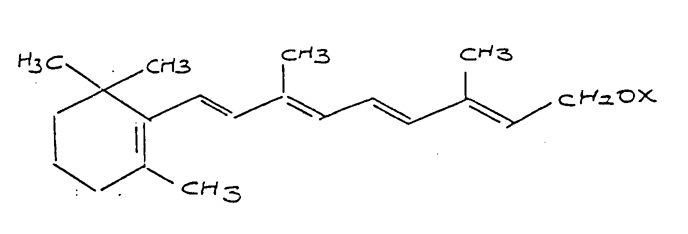
where X represents H or -COR where R represents a group chosen from branched or unbranched, alkyl or alkenyl groups having an average of from 1 to 20 carbon atoms
for topical application to human skin to reduce or prevent the damaging effects of UV light on skin and/or to lighten the skin,
   wherein the amount of skin lightening agent and the amount of retinol or the derivative thereof are expressed with respect to the total composition and wherein the composition is free of cholecalciferol derivative.

The invention also provides the use of an effective amount of from 0.01 to 20% by weight of a skin lightening agent chosen from L-ascorbic acid and derivatives thereof, kojic acid and derivatives thereof, hydroquinone and derivatives thereof, extract of placenta, arbutin, niacin, niacinamide, α hydroxy acids, phloretin, phloridzin, liquorice extract, cysteaminylphenol and derivatives thereof and compounds having the structure (2): 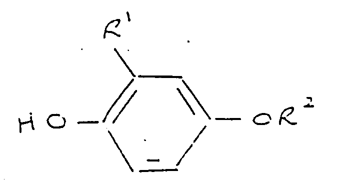
where R¹ represents H, or an ether group represented by OR³,
R² and R³ are the same or different and each represents a group chosen from branched or unbranched alkyl or alkenyl groups having an average of from 1 to 20 carbon atoms,
in the preparation of a composition for topical application to human skin, which Composition comprises an effective amount of from 0.001 to 10% by weight of retinol or a derivative thereof having the structure (1): 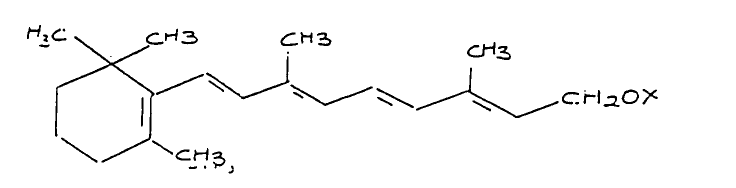
where X represents H or -COR where R represents a group chosen from branched or unbranched, alkyl or alkenyl groups having an average of from 1 to 20 carbon atoms
for the purpose of promoting repair of photo-damaged skin and/or of reducing or preventing the damaging effects of ultra violet light on skin,
   wherein the amount of skin lightening agent and the amount of retinol or the derivative thereof are expressed with respect to the total composition and wherein the composition is free of cholecalciferol derivative.

### DISCLOSURE OF THE INVENTION

Invention concerns the use of a combination of retinol or a derivative thereof together with a skin lightening agent which together behave synergistically in lightening skin and reducing skin blotchiness and mottling due to hyperpigmentation. Furthermore, due to the rejuvenating influence of the retinol or its derivative on skin, there will be an overall improvement in skin texture with reduction in fine wrinkling and improved skin colour. Also, co-formulation with a sunscreen will enhance the photo stability and activity of retinol or its derivative within the formulation and also prevent further actinic damage to the skin.

### Retinol and derivatives thereof

The invention involves the use of retinol or a derivative thereof having the structure (1).

In addition to retinol itself, examples of derivatives of retinol include:
Retinyl acetate
Retinyl butyrate
Retinyl propionate
Retinyl octanoate
Retinyl laurate
Retinyl palmitate
Retinyl oleate
Retinyl linoleate.

The amount of retinol, or a derivative thereof, present in the composition is from 0.001 to 10% and preferably 0.01 to 5% by weight of the composition.

Preferably the composition comprises retinol, most preferably the composition comprises the trans-isomer of retinol.

### The skin lightening agent

The invention also involves the use of a skin lightening agent in the composition.

The skin lightening agent is chosen from:
L-ascorbic acid, and derivatives thereof
Kojic acid, and derivatives thereof
Hydroquinone and derivatives thereof
Extract of placenta
Compounds having the structure (2)
Arbutin
α hydroxy acids
phloretin
phloridzin,
liquorice extract
cysteaminylphenol and derivatives thereof
Niacin, and
Niacinamide.

Preferably the skin lightening agent is hydroquinone and derivatives thereof, most preferably hydroquinone.

The amount of skin lightening agent which is used in the composition is from 0.01 to 20%, preferably 0.1 to 10% by weight of the composition.

### The Cosmetically Acceptable Vehicle

The composition also comprises a cosmetically acceptable vehicle to act as a dilutant, dispersant or carrier for other materials present in the composition, so as to facilitate their distribution when the composition is applied to the skin.

Vehicles other than water can include liquid or solid emollients, solvents, humectants, thickeners and powders. Examples of each of these types of vehicle, which can be used singly or as mixtures of one or more vehicles, are as follows:

Emollients, such as stearyl alcohol, glyceryl monoricinoleate, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, eicosanyl alcohol, behenyl alcohol, cetyl palmitate, silicone oils such as dimethylpolysiloxane, di-n-butyl sebacate, isopropyl myristate, isopropyl palmitate, isopropyl stearate, butyl stearate, polyethylene glycol, triethylene glycol, lanolin, cocoa butter, corn oil, cotton seed oil, olive oil, palm kernel oil, rapeseed oil, safflower seed oil, evening primrose oil, soybean oil, sunflower seed oil, avocado oil, sesame seed oil, coconut oil, arachis oil, castor oil, acetylated lanolin alcohols, petroleum jelly, mineral oil, butyl myristate, isostearic acid, palmitic acid, isopropyl linoleate, lauryl lactate, myristyl lactate, decyl oleate, myristyl myristate;

Propellants, such as propane, butane, isobutane, dimethyl ether, carbon dioxide, nitrous oxide;

Solvents, such as ethyl alcohol, isopropanol, acetone, ethylene glycol monoethyl ether, diethylene glycol monobutyl ether, diethylene glycol monoethyl ether;

Powders, such as chalk, talc, fullers earth, kaolin, starch, gums, colloidal silica sodium polyacrylate, tetra alkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose, ethylene glycol monostearate.

The cosmetically acceptable vehicle will usually form from 10 to 99.9%, preferably from 50 to 99% by weight of the emulsion, and can, in the absence of other cosmetic adjuncts, form the balance of the emulsion.

### Organic sunscreens

The composition optionally can comprise an organic sunscreen further to enhance the benefit of the composition in providing protection from the harmful effects of excessive exposure to sunlight.

Examples of suitable organic sunscreens, when required, include those set out in Table 1 below, and mixtures thereof.

**TABLE 1**

| CTFA Name | Trade Name | Supplier |
|---|---|---|
| Benzophenone-3 | UVINUL® M-40 | BASF Chemical Co. |
| Benzophenone-4 | UVINUL® MS-40 | BASF Chemical Co. |
| Benzophenone-8 | SPECRA-SORB® UV-24 | American Cyanamide |
| DEA Methoxycinnamate | BERNEL HYDRO® | Bernel Chemical |
| Ethyl dihydroxypropyl-PABA | AMERSCREEN® P | Amerchol Corp. |
| Glyceryl PABA | NIPA® G.M.P.A. | Nipa Labs. |
| Homosalate | KEMESTER® HMS | Hunko Chemical |
| Methyl anthranilate | SUNAROME® UVA | Felton Worldwide |
| Octocrylene | UVINUL® N-539 | BASF Chemical Co. |
| Octyl dimethyl PABA | AMERSCOL® | Amerchol Corp. |
| Octyl methoxycinnamate | PARSOL® MCX | Bernel Chemical |
| Octyl salicylate | SUNAROME® WMO | Felton Worldwide |
| PABA | PABA® | National Starch |
| 2-Phenylbenzimidazole-5-sulphonic acid | EUSOLEX® 232 | EM Industries |
| TEA salicylate | SUNAROME® W | Felton Worldwide |
| 3-(4-methylbenzylidene)-camphor | EUSOLEX® 6300 | EM Industries |
| Benzophenone-1 | UVINUL® 400 | BASF Chemical Co. |
| Benzophenone-2 | UVINUL® D-50 | BASF Chemical Co. |
| Benzophenone-6 | UVINUL® D-49 | BASF Chemical Co. |
| Benzophenone-12 | UVINUL® 408 | BASF Chemical Co. |
| 4-Isopropyl dibenzoyl methane | EUSOLEX® 8020 | EM Industries |
| Butyl methoxy dibenzoyl methane | PARSOL® 1789 | Givaudan Corp. |
| Etocrylene | UVINUL® | BASF Chemical Co. |

The composition of the invention can accordingly comprise from 0.1 to 10%, preferably from 1 to 5% by weight of an Organic sunscreen material.

### Inorganic sunscreen

The composition optionally can also comprise as a sunscreen ultrafine titanium dioxide in either of two forms, namely water-dispersible titanium dioxide and oil-dispersible titanium dioxide.

Water-dispersible titanium dioxide is ultrafine titanium dioxide, the particles of which are uncoated or which are coated with a material to impart a hydrophilic surface property to the particles. Examples of such materials include aluminium oxide and aluminium silicate.

Oil-dispersible titanium dioxide is ultrafine titanium dioxide, the particles of which exhibit a hydrophobic surface property, and which, for this purpose, can be coated with metal soaps such as aluminium stearate, aluminium laurate or zinc stearate, or with organosilicone compounds.

By "ultrafine titanium dioxide" is meant particles of titanium dioxide having an average particle size of less than 100nm, preferably from 10 to 40nm and most preferably from 15 to 25nm.

By topical application to the skin of a mixture of both water-dispersible ultrafine titanium dioxide and oil-dispersible ultrafine titanium dioxide, synergistically enhanced protection of the skin against the harmful effects of both UV-A and UV-B rays is achievable.

It is believed that this unexpected benefit is due to the deposition of each type of titanium dioxide on different regions of the skin surface, water-dispersible titanium dioxide being preferentially retained by hydrophilic regions of the skin's surface, while oil-dispersible titanium dioxide is retained preferentially by hydrophobic regions of the skin's surface. The combined overall effect is that more efficient physical coverage of the skin's surface is attainable and this can be demonstrated by measurement of the Sun Protection Factor (SPF).

In order to achieve the enhanced, synergistic benefit, as herein described, the weight ratio of water-dispersible titanium dioxide to oil-dispersible titanium dioxide should be from 1:4 to 4:1, preferably from 1:2 to 2:1 and ideally about equal weight proportions.

The total amount of titanium dioxide that can optionally can be incorporated in the composition according to the invention is from 1 to 25%, preferably from 2 to 10% and ideally from 3 to 7% by weight of the composition.

### Other Inorganic Sunscreens

The emulsion optionally can comprise an inorganic sunscreen in addition to ultrafine titanium dioxide as herein defined.

Examples of other inorganic sunscreens include:
zinc oxide, having an average particle size of from 1 to 300nm,
iron oxide, having an average particle size of from 1 to 300nm,
silica, such as fumed silica, having an average particle size of from 1 to 100nm.

It should be noted that silica, when used as an ingredient in the emulsion according to the invention can provide protection from infrared radiation.

### OPTIONAL SKIN BENEFIT MATERIALS AND COSMETIC ADJUNCTS

A particularly convenient form of the composition is an emulsion, in which case an oil or oily material will normally be present, together with an emulsifier to provide either a water-in-oil emulsion or an oil-in-water emulsion, depending largely on the average hydrophilic-lyophilic balance (HLB) of the emulsifier employed.

### Oil or oily material

The composition can optionally comprise one or more oils or other materials having the properties of an oil.

Examples of suitable oils include mineral oil and vegetable oils, and oil materials, such as those already proposed herein as emollients. Other oils or oily materials include silicone oils, both volatile and non-volatile, such as polydimethyl siloxanes.

The oil or oily material, when present for the purposes for forming an emulsion, will normally form up to 90%, preferably from 10 to 80% by volume of the composition.

### Emulsifier

The composition can also optionally comprise one or more emulsifiers the choice of which will normally determine whether a water-in-oil or and oil-in-water emulsion is formed.

When a water-in-oil emulsion is required, the chosen emulsifier or emulsifiers should normally have an average HLB value of from 1 to 6. When an oil-in-water emulsion is required, a chosen emulsifier or emulsifiers should have an average HLB value of >6.

Examples of suitable emulsifiers are set below in Table 1 in which the chemical name of the emulsifiers is given together with an example of a trade name as commercially available, and the average HLB value.

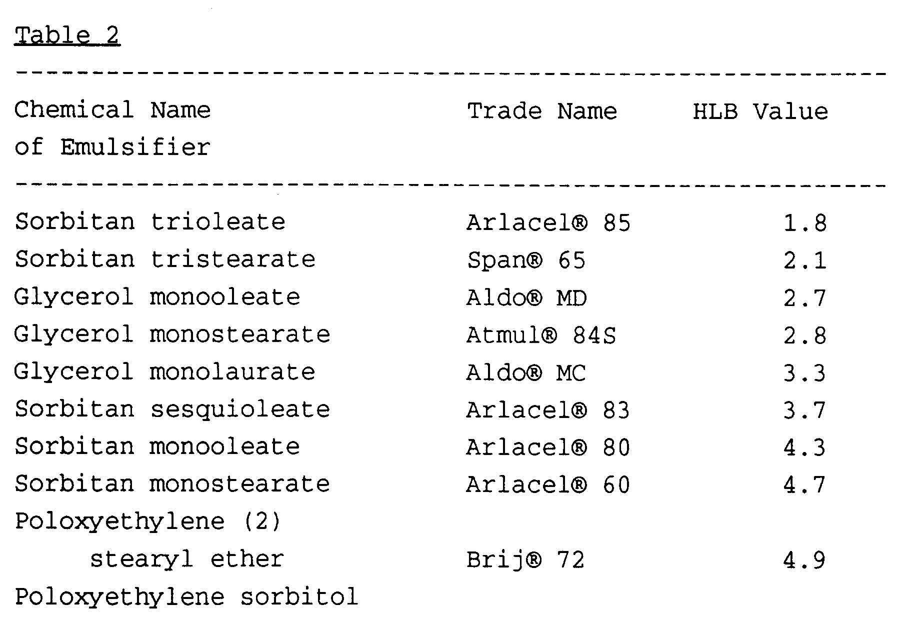

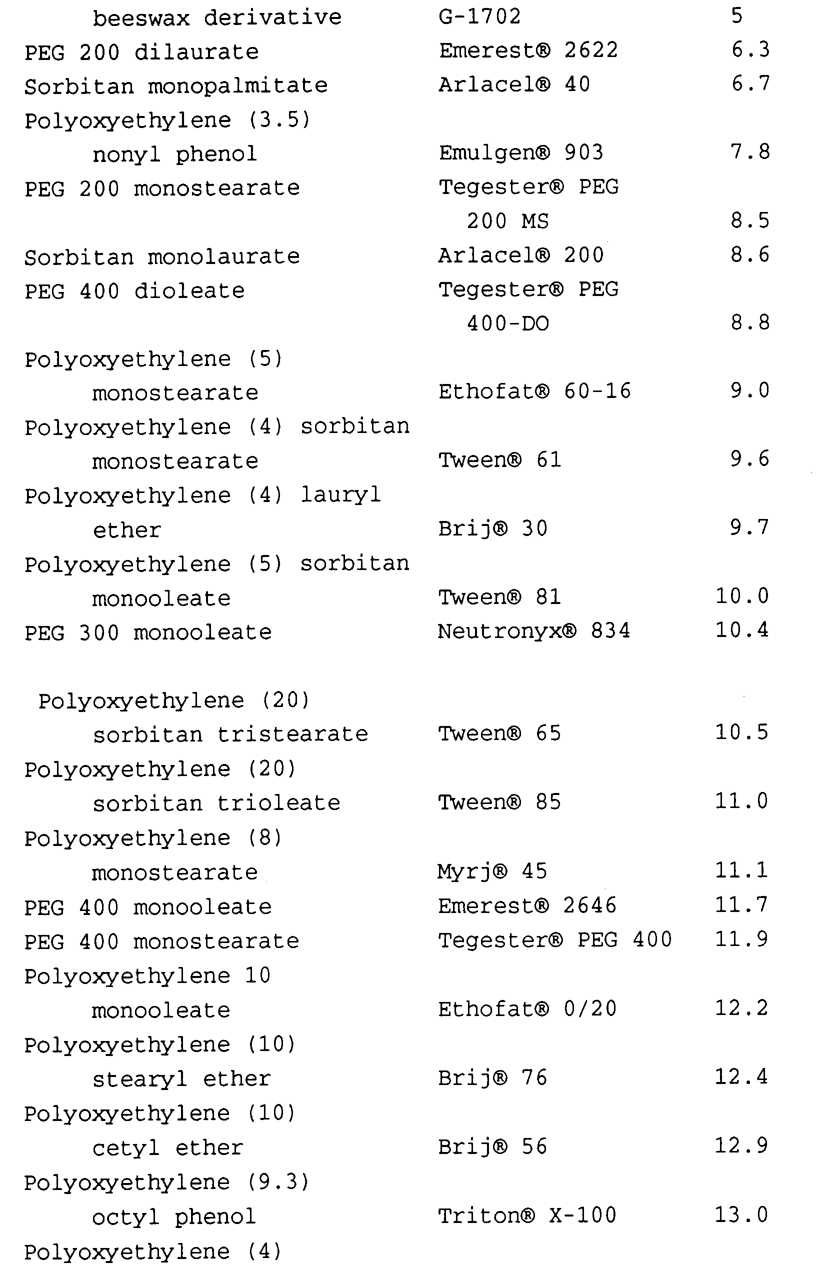

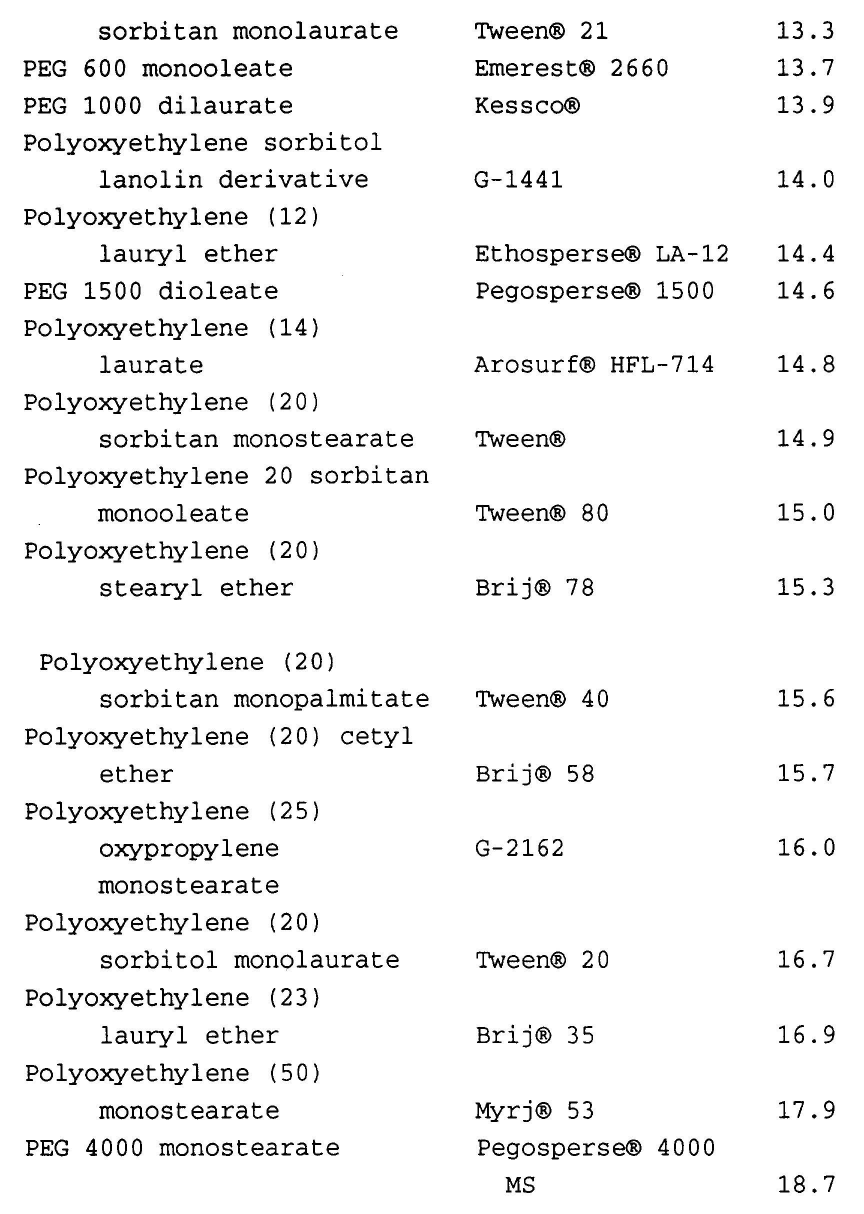

It is to be understood that two or more emulsifiers can be employed if desired.

The amount of emulsifier or mixtures thereof, that optionally can be incorporated in the composition is from 1 to 50%, preferably from 2 to 20% and most preferably from 2 to 10% by weight of the composition.

### Water

The composition can an also comprise water, usually up to 80%, preferably from 5 to 80% by volume.

### Silicone Surfactant

The composition can also optionally comprise a high molecular weight silicone surfactant which can also act as an emulsifier, in place of or in addition to the optional emulsifier(s) already mentioned.

The silicone surfactant is a high molecular weight polymer of dimethyl polysiloxane with polyoxyethylene and/or polyoxypropylene side chains having a molecular weight of from 10,000 to 50,000 and having the structure: 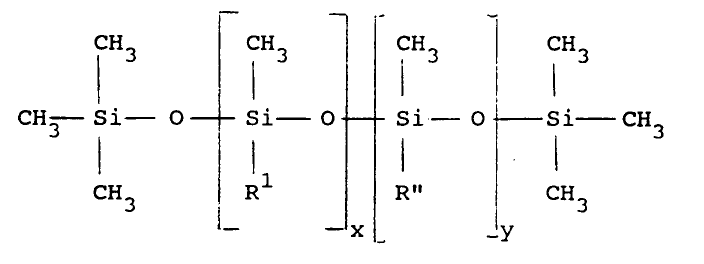 where the groups R' and R'' are each chosen from -H, C₁₋₁₈ alkyl and 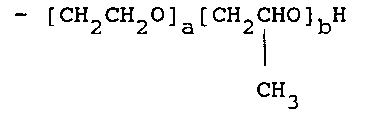
a has a value of from 9 to 115,
b has a value of from 0 to 50,
x has a value of from 133 to 673,
y has a value of from 25 to 0.25.

Preferably, the dimethyl polysiloxane polymer is one in which:
a has a value of from 10 to 114
b has a value of from 0 to 49
x has a value of from 388 to 402
y has a value of from 15 to 0.75
one of groups R' and R'' being lauryl, and the other having a molcular weight of from 1000 to 5000.

A particularly preferred dimethyl polysiloxane polymer is one in which:
a has the value 14
b has the value 13
x has the value 249
y has the value 1.25

The dimethyl polysiloxane polymer is conveniently provided as a dispersion in a volatile siloxane, the dispersion comprising, for example, from 1 to 20% by volume of the polymer and from 80 to 99% by volume of the volatile siloxane. Ideally, the dispersion consists of a 10% by volume of the polymer dispersed in the volatile siloxane.

Examples of the volatile siloxanes in which the polysiloxane polymer can be dispersed include polydimethyl siloxane (pentamer and/or hexamer).

A particularly preferred silicone surfactant is cyclomethicone and dimethicone copolyol, such as DC 3225C Formulation Aid available from DOW CORNING. Another is laurylmethicone copolyol, such as DC Q2-5200, also available from Dow Corning.

The amount of silicone surfactant, when present in the composition will normally be up to 25%, preferably from 0.5 to 15% by weight of the emulsion.

### Other Cosmetic Adjuncts

Examples of conventional adjuncts which can optionally be employed include preservatives, such as para-hydroxy benzoate esters; antioxidants, such butyl hydroxy toluene; humectants, such as glycerol, sorbitol, 2-pyrrolidone-5-carboxylate, dibutylphthalate, gelatin, polyethylene glycol, such as PEG 200-600; buffers, such as lactic acid together with a base such as triethanolamine or sodium hydroxide; waxes, such as beeswax, ozokerite wax, paraffin wax; plant extracts, such as Aloe vera, cornflower, witch hazel, elderflower, cucumber; thickeners; activity enhancers; colourants; and perfumes. Cosmetic adjuncts can form the balance of the composition.

### Use of the Composition

The composition is intended primarily as a skin care product for topical application to human skin to lighten skin and/or to repair photo-damaged skin and to prevent photo-damage to skin due to exposure to sunlight. In particular, the composition can be used to reduce skin blotchiness and mottling due to hyperpigmentation, to improve skin texture with reductions in fine wrinkling and otherwise to improve skin colour. In general, the composition, when topically applied to skin, is useful in the prevention of actinic damage to all epidermal cells.

In use, a small quantity of the composition, for example from 1 to 5ml, is applied to exposed areas of the skin, from a suitable container or applicator and, if necessary, it is then spread over and/or rubbed into the skin using the hand or fingers or a suitable device.

### PRODUCT FORM AND PACKAGING

The topical skin treatment composition can be formulated as a lotion having a viscosity of from 4,000 to 10,000 mPas, a fluid cream having a viscosity of from 10,000 to 20,000 mPas or a cream having a viscosity of from 20,000 to 100,000 mPas, or above. The composition can be packaged in a suitable container to suit its viscosity and intended use by the consumer.

For example, a lotion or fluid cream can be packaged in a bottle or a roll-ball applicator or a propellant-driven aerosol device or a container fitted with a pump suitable for finger operation. When the composition is a cream, it can simply be stored in a non-deformable bottle or squeeze container, such as a tube or a lidded jar.

### Test method - In vitro melanocyte cell culture

Pigment producing cells derived from a mammalian melanoma are grown in culture by standard methods. Preferred cell lines are B16 or S-91 cells, but other lines or primary mouse or human melanocytes can be used.

Melanoma cells are grown in cell culture medium such as RPMI 1640 (GIBCO) supplemented with fetal calf serum and glutamine to approximately 1/3 confluence. The active is dissolved in culture medium, the pH adjusted as required and sterile filtered. The solution is then added to the cells.

The cells are cultured for a further period of 4 days and the amount of melanin produced assayed by measuring the absorbance at 540 nm of the melanin released into the medium.

Cell viability is tested using neutral red (3-amino-7-dimethylamino-2-methyl phenazine hydrochloride) a water soluble vital dye which passes through the intact plasma membrane and becomes concentrated in lysosomes of viable cells. For any culture, the amount of dye taken up is proportional to the number of viable cells and agents that damage cell and lysosomal membranes inhibit dye incorporation.

The cells are incubated in 50 µg/ml neutral red solution for 3 hours at 37°C in 5% CO₂ in air. The solution is aspirated, the cells washed once in saline and to them added a solvent (50% H₂O, 49% ethanol, 1% acetic acid) to solubilise the dye. The amount of neutral red dye is quantified by measuring absorbance at 540 nm.

### Results

The above procedure was used to assess the ability of compositions of skin lightening agents (at a range of concentrations) and retinol (at a range of concentrations) to reduce the amount of melanin produced without affecting cell viability.

These compositions were compared with compositions having retinol alone at a range of concentrations and skin lightening agents alone at a range of concentrations.

The results for both viability and melanin production were calculated as percentages of the control which contained medium alone. Results are given in Table 3 and 4.

Results clearly show that the skin lightening agents and retinol act synergistically to reduce melanin production. There were no effects on cell viability at these concentrations.

**Table 3**

| Trans retinol (µm) | Hydroquinone (mM) | | | |
|---|---|---|---|---|
| | 0 | 0.001 | 0.0025 | 0.005 |
| 0 | 100 | 101.1 | 100.1 | 49.8* |
| 2.0 | 115.3* | 69.3* | 40.7* | 15.8* |
| 6.7 | 87.5* | 15.1* | 14.8* | 10.1* |

| | | | | |
|---|---|---|---|---|
| * percentage is significantly different (P<0.05) from control. | | | | |

**Table 4**

| Trans Retinol (M) | Kojic Acid (mM) | | |
|---|---|---|---|
| | 0.01 | 0.1 | 1 |
| 10⁻⁶ | 78 | 73 | 8 |

### EXAMPLES

The invention is further illustrated by the following examples; in each formulation, the titanium dioxide employed was ultrafine titanium dioxide having a mean particle size of from 15 to 25nm.

### Example 1

This example illustrates a lotion to be used according to the invention.

| Ingredient | % w/w |
|---|---|
| retinyl propionate | 1 |
| kojic acid | 1 |
| silicone surfactant | 10 |
| volatile siloxane | 14 |
| mineral oil | 1.5 |
| titanium dioxide (water-dispersible) | 2.5 |
| titanium dioxide (oil-dispersible) | 2.5 |
| 2-hydroxy octanoic acid | 1 |
| 2-hydroxy propanoic acid | 5 |
| butylene glycol | 10 |
| sodium chloride | 2 |
| l-proline | 0.1 |
| neutralising agent | qs |
| preservative | qs |
| perfume | qs |
| water | qs |

### Example 2

This example illustrates a fluid cream to be used according to the invention.

| Ingredient | % w/w |
|---|---|
| retinyl acetate | 0.3 |
| niacinamide | 1 |
| volatile siloxane (DC 345) | 8.2 |
| silicone surfactant (DC 3225C) | 12 |
| petroleum jelly | 0.5 |
| mineral oil | 1.5 |
| Parsol MCX (octyl methoxycinnamate) | 3 |
| titanium dioxide (oil-dispersible) | 2 |
| titanium dioxide (water-dispersible) | 2 |
| sodium chloride | 2 |
| butylene glycol | 10 |
| l-proline | 0.1 |
| 2-hydroxy octanoic acid | 1 |
| 2-hydroxy propanoic acid | 5 |
| neutralising agent | qs |
| preservative | qs |
| perfume | qs |
| water | qs |

### Example 3

This example illustrates a cream to be used according to the invention.

| Ingredient | % w/w |
|---|---|
| retinyl palmitate | 1 |
| L-ascorbic acid | 2 |
| volatile siloxane (DC 345 Fluid) | 8.2 |
| silicone surfactant (DC 3225C) | 12 |
| mineral oil | 1.5 |
| petroleum jelly | 0.5 |
| Parsol MCX (octyl methoxycinnamate) | 1.5 |
| titanium dioxide (oil-dispersible) | 1.0 |
| titanium dioxide (water-dispersible) | 1 |
| 2-hydroxyoctanoic acid | 1 |
| 2-hydroxypropanoic acid | 5 |
| sodium chloride | 2 |
| butylene glycol | 10 |
| l-proline | 0.1 |
| neutralising agent (aqueous phase to 4.5) | q.s. |
| preservative | q.s. |
| perfume | q.s. |
| water | to 100 |

### Example 4

This example illustrates a lotion to be used according to the invention.

| Ingredient | % w/w |
|---|---|
| retinyl linoleate | 0.5 |
| retinyl palmitate | 0.5 |
| arbutin | 2 |
| silicone surfactant (DC 3225C) | 10 |
| volatile siloxane (DC 345) | 14 |
| mineral oil | 1.5 |
| Parsol MCX | 3 |
| titanium dioxide (oil-dispersible) | 2 |
| titanium dioxide (water-dispersible) | 2 |
| butylene glycol | 10 |
| sodium chloride | 2 |
| l-proline | 0.1 |
| 2-hydroxy octanoic acid | 1 |
| 2-hydroxy propanoic acid | 5 |
| neutralising agent | qs |
| perfume | qs |
| preservative | qs |
| water | qs |

### Example 5

This example illustrates a sunscreen cream to be used in accordance with the invention.

| Ingredient | % w/w |
|---|---|
| retinyl oleate | 2 |
| retinyl acetate | 1 |
| hydroquinone | 1 |
| Polyoxyethylene (2) stearyl alcohol | 3 |
| Polyoxyethylene (21) stearyl alcohol | 2 |
| cetyl alcohol | 1.5 |
| soft white paraffin | 1.5 |
| silicone fluid 200 | 5 |
| liquid paraffin | 8 |
| glycerin | 2 |
| preservatives | 0.5 |
| titanium dioxide (water-dispersible) | 2.5 |
| titanium dioxide (oil-dispersible) | 2.5 |
| water | to 100 |

### Example 6

This example also illustrates a sunscreen cream to be used in accordance with the invention.

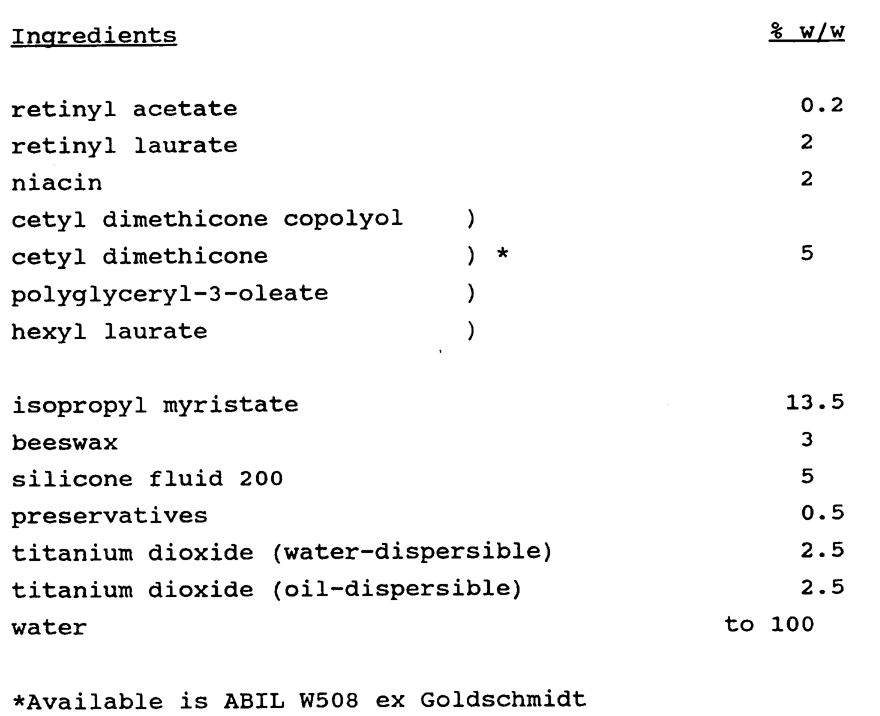

### Example 7

This example illustrates a lotion to be used according to the invention.

| Ingredient | % w/w |
|---|---|
| retinyl octanoate | 2 |
| phloretin | 2 |
| silicone surfactant | 10 |
| volatile siloxane | 14 |
| mineral oil | 1.5 |
| ultrafine titanium dioxide (water-dispersible) | 5 |
| 2-hydroxy octanoic acid | 1 |
| 2-hydroxy propanoic acid | 5 |
| butylene glycol | 10 |
| sodium chloride | 2 |
| amino acid | 0.1 |
| neutralising agent | qs |
| preservative | qs |
| perfume | qs |
| water | qs |

### Example 8

This example illustrates a lotion to be used according to the invention.

| Ingredient | % w/w |
|---|---|
| retinyl palmitate | 2 |
| hydroquinone monomethyl ether | 0.5 |
| silicone surfactant | 10 |
| volatile siloxane | 14 |
| mineral oil | 1.5 |
| ultrafine titanium dioxide (oil-dispersible) | 5 |
| 2-hydroxy octanoic acid | 1 |
| 2-hydroxy propanoic acid | 5 |
| butylene glycol | 10 |
| sodium chloride | 2 |
| amino acid | 0.1 |
| neutralising agent | qs |
| preservative | qs |
| perfume | qs |
| water | qs |

### Example 9

This example illustrates a lotion to be used according to the invention.

| Ingredient | % w/w |
|---|---|
| retinyl octanoate | 1 |
| retinyl linoleate | 1 |
| phloridzin | 1 |
| silicone surfactant | 10 |
| volatile siloxane | 14 |
| mineral oil | 1.5 |
| ultrafine titanium dioxide (water-dispersible) | 2.5 |
| ultrafine titanium dioxide (oil-dispersible) | 2.50 |
| 2-hydroxy octanoic acid | 1 |
| 2-hydroxy propanoic acid | 5 |
| butylene glycol | 10 |
| sodium chloride | 2 |
| amino acid | 0.1 |
| neutralising agent | qs |
| preservative | qs |
| perfume | qs |
| water | qs |

### Example 10

This example illustrates an anhydrous formulation to be used in accordance with the invention.

| Ingredient | % w/w |
|---|---|
| retinol | 0.2 |
| 4-S-cysteaminylphenol | 1 |
| Iso-propyl alcohol | 10 |
| volatile silicone | 80 |
| ethyl hexyl palmitate | 8.7 |
| antioxidant | 0.1 |

## Claims

1. Use of an effective amount of from 0.01 to 20% by weight of a skin lightening agent chosen from L-ascorbic acid and derivatives thereof, kojic acid and derivatives thereof, hydroquinone and derivatives thereof, extract of placenta, arbutin, niacin, niacinamide, α hydroxy acids, phloretin, phloridzin, liquorice extract, cysteaminylphenol and derivatives thereof and compounds having the structure (2): 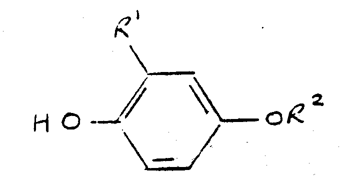
where R¹ represents H, or an ether group represented by OR³,
R² and R³ are the same or different and each represents a group chosen from branched or unbranched alkyl or alkenyl groups having an average of from 1 to 20 carbon atoms,
in a cosmetic composition which composition comprises an effective amount of from 0.001 to 10% by weight of retinol or a derivative thereof having the structure (1): 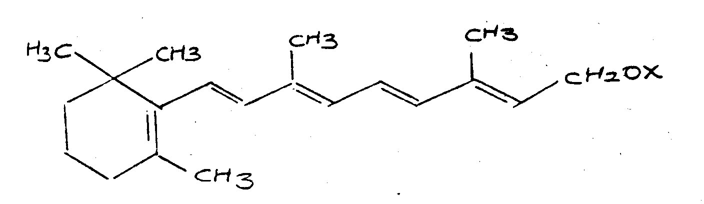
where X represents H or -COR where R represents a group chosen from branched or unbranched, alkyl or alkenyl groups having an average of from 1 to 20 carbon atoms
for topical application to human skin to reduce or prevent the damaging effects of UV light on skin and/or to lighten the skin,
wherein the amount of skin lightening agent and the amount of retinol or the derivative thereof are expressed with respect to the total composition and wherein the composition is free of cholecalciferol derivative.

2. Use of an effective amount of from 0.01 to 20% by weight of a skin lightening agent chosen from L-ascorbic acid and derivatives thereof, kojic acid and derivatives thereof, hydroquinone and derivatives thereof, extract of placenta, arbutin, niacin, niacinamide, α hydroxy acids, phloretin, phloridzin, liquorice extract, cysteaminylphenol and derivatives thereof and compounds having the structure (2): 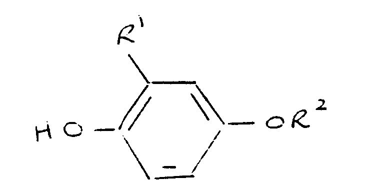
where R¹ represents H, or an ether group represented by OR³,
R² and R³ are the same or different and each represents a group chosen from branched or unbranched alkyl or alkenyl groups having an average of from 1 to 20 carbon atoms,
in the preparation of a composition for topical application to human skin, which composition comprises an effective amount of from 0.001 to 10% by weight of retinol or a derivative thereof having the structure (1): 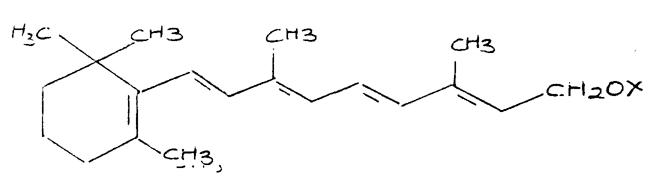
where X represents H or -COR where R represents a group chosen from branched or unbranched, alkyl or alkenyl groups having an average of from 1 to 20 carbon atoms
for the purpose of promoting repair of photo-damaged skin and/or of reducing or preventing the damaging effects of ultra violet light on skin,
wherein the amount of skin lightening agent and the amount of retinol or the derivative thereof are expressed with respect to the total composition and wherein the composition is free of cholecalciferol derivative.

3. Use according to claim 1 or claim 2 wherein the amount of retinol or a derivative thereof having the structure (1) is from 0.01 to 5% by weight of the composition.

4. Use according to any preceding claim wherein the amount the skin lightening agent is from 0.1 to 10% by weight of the composition.

5. Use according to any preceding claim wherein the skin lightening agent is hydroquinone or a derivative thereof.

6. Use according to any preceding claim wherein the skin lightening agent is hydroquinone.

7. Use according to any preceding claim wherein the composition comprises trans-retinol.

## Patentansprüche

1. Verwendung einer wirksamen Menge von 0,01 bis 20 Gew.-% eines Hautaufhellungsmittels, ausgewählt aus L-Ascorbinsäure und Derivaten davon, Koji-säure und Derivaten davon, Hydrochinon und Derivaten davon, Plazentaextrakt, Arbutin, Niacin, Niacinamid, α-Hydroxysäuren, Phloretin, Phlorizin, Süßholzextrakt, Cysteaminylphenol und Derivaten davon, und Verbindungen mit der Strukturformel (2): 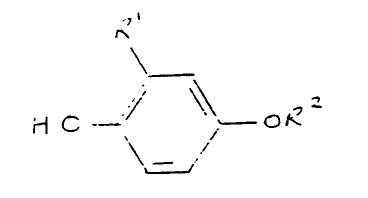
worin R¹ H oder eine Ethergruppe OR³ bedeutet,
R² und R³ gleich oder verschieden sind und jeweils eine Gruppe bedeuten, ausgewählt aus verzweigten oder unverzweigten Alkyl- oder Alkenylgruppen mit durchschnittlich 1 bis 20 Kohlenstoffatomen,
in einer kosmetischen Zusammensetzung, wobei die Zusammensetzung eine wirksame Menge von 0,001 bis 10 Gew.-% Retinol oder einem Derivat davon mit der Strukturformel (1): 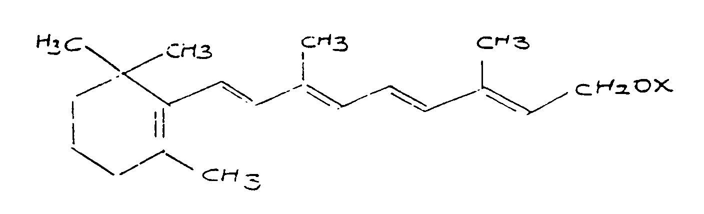
enthält, worin X H oder -COR bedeutet, worin R eine Gruppe bedeutet, ausgewählt aus verzweigten oder unverzweigten Alkyl- oder Alkenylgruppen mit durchschnittlich 1 bis 20 Kohlenstoffatomen,
für die topische Behandlungen von menschlicher Haut, um die schädigenden Wirkungen von UV-Licht auf Haut zu vermindern oder zu verhindern und/oder die Haut aufzuhellen,
wobei die Menge an Hautaufhellungsmittel und die Menge an Retinol oder einem Derivat davon auf die Zusammensetzung bezogen ausgedrückt werden und wobei die Zusammensetzung frei von Cholecalciferolderivaten ist.

2. Verwendung einer wirksamen Menge von 0,01 bis 20 Gew.-% eines Hautaufhellungsmittels, ausgewählt aus L-Ascorbinsäure und Derivaten davon, Koji-säure und Derivaten davon, Hydrochinon und Derivaten davon, Plazentaextrakt, Albutin, Niacin, Niacinamid, α-Hydroxysäuren, Phloretin, Phlorizin, Süßholzextrakt, Cysteaminylphenol und Derivaten davon, und Verbindungen mit der Strukturformel (2): 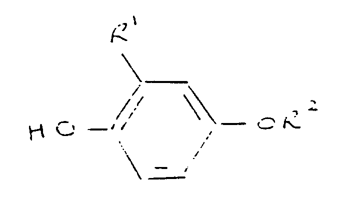
worin R¹ H oder eine Ethergruppe OR³ bedeutet,
R² und R³ gleich oder verschieden sind und jeweils eine Gruppe bedeuten, ausgewählt aus verzweigten oder unverzweigten Alkyl- oder Alkenylgruppen mit durchschnittlich 1 bis 20 Kohlenstoffatomen,
zur Herstellung einer kosmetischen Zusammensetzung, für die topische Behandlung menschlicher Haut, wobei die Zusammensetzung eine wirksame Menge von 0,001 bis 10 Gew.% Retinol oder einem Derivat davon mit der Strukturformel (1): 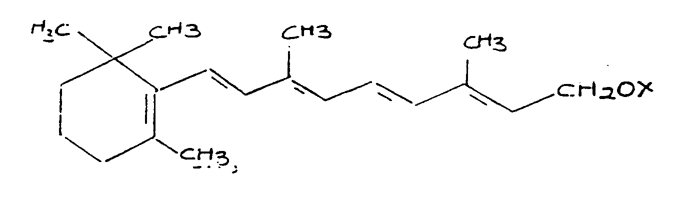
enthält, worin X H oder -COR bedeutet, worin R eine Gruppe bedeutet, ausgewählt aus verzweigten oder unverzweigten Alkyl- oder Alkenylgruppen mit durchschnittlich 1 bis 20 Kohlenstoffatomen,
um die Reparatur von lichtgeschädigter Haut zu fördern und/oder die schädigenden Wirkungen von Ultraviolettlicht auf die Haut zu vermindern oder zu verhindern,
wobei die Menge an Hautaufhellungsmittel und die Menge an Retinol oder eines Derivats davon in bezug auf die Gesamtzusammensetzung ausgedrückt werden, worbei die Zusammensetzung frei von Cholecalciferolderivaten ist.

3. Verwendung nach Anspruch 1 oder 2, worin die Menge an Retinol oder einem Derivat davon mit der Strukturformel (1) zwischen 0,01 und 5 Gew.-% der Zusammensetzung liegt.

4. Verwendung nach einem der vorhergehenden Ansprüche, worin die Menge des Hautaufhellungmittels zwischen 0,1 und 10 Gew.-%, bezogen auf die Zusammensetzung, liegt.

5. Verwendung nach einem der vorhergehenden Ansprüche, worin das Hautaufhellungsmittel Hydrochinon oder ein Derivat davon ist.

6. Verwendung nach einem der vorgehenden Ansprüche, worin das Hautaufhellungsmittel Hydrochinon ist.

7. Verwendung nach einem der vorgehenden Ansprüche, worin die Zusammensetzung Transretinol enthält.

## Revendications

1. Utilisation d'une quantité efficace allant de 0,01 à 20 % en masse d'un agent d'éclaircissement de la peau choisi parmi l'acide L-ascorbique et ses dérivés, l'acide cojique et ses dérivés, l'hydroquinone et ses dérivés, un extrait de placenta, de l'arbutine, de la niacine, du niacinamide, des acides α-hydroxy, de la phlorétine, de la phloridzine, un extrait de réglisse, du cystéaminylphénol et ses dérivés et des composés ayant la structure (2) suivante : 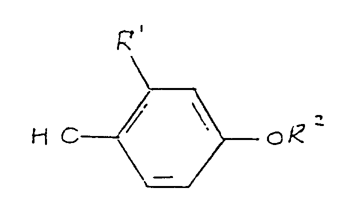
où R¹ représente H ou un groupe éther représenté par OR³,
R² et R³ sont identiques ou différents et chacun représente un groupe choisi parmi des groupes alkyle ou alkényle ramifiés ou non ramifiés présentant une moyenne de 1 à 20 atome(s) de carbone,
dans une composition cosmétique, laquelle composition comprend une quantité efficace allant de 0,001 à 10 % en masse de rétinol ou d'un dérivé de celui-ci dont la structure (1) est la suivante : 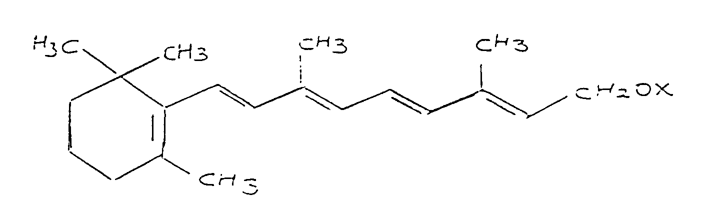
ou X représente H ou -COR, où R représente un groupe choisi parmi des groupes alkyle ou alkényle ramifiés ou non ramifiés présentant une moyenne de 1 à 20 atome(s) de carbone,
pour l'application topique sur la peau humaine afin de réduire ou de prévenir les effets nuisibles du rayonnement U.V. sur la peau et/ou d'éclaircir la peau,
dans laquelle la quantité d'agent d'éclaircissement de la peau et la quantité de rétinol ou de son dérivé sont exprimées respectivement à la composition totale et dans laquelle la composition est exempte de dérivé de cholécalciférol.

2. Utilisation d'une quantité efficace comprise entre 0,01 et 20 % en masse d'un agent d'éclaircissement de la peau choisi parmi l'acide L-ascorbique et ses dérivés, l'acide cojique et ses dérivés, l'hydroquinone et ses dérivés, un extrait de placenta, de l'arbutine, de la niacine, du niacinamide, des acides α-hydroxy, de la phlorétine, de la phloridzine, un extrait de réglisse, du cystéaminylphénol et ses dérivés et des composés ayant la structure (2) suivante : 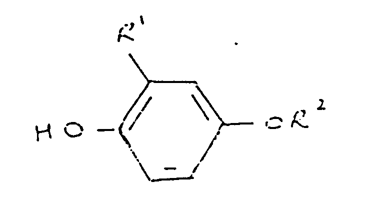
où R¹ représente H ou un groupe éther représenté par OR³,
R² et R³ sont identiques ou différents et chacun représente un groupe choisi parmi des groupes alkyle ou alkényle ramifiés ou non ramifiés présentant une moyenne de 1 à 20 atome(s) de carbone,
dans la préparation d'une composition pour application topique sur la peau humaine, laquelle composition comprend une quantité efficace allant de 0,001 à 10 % en masse de rétinol ou d'un dérivé de celui-ci dont la structure (1) est la suivante : 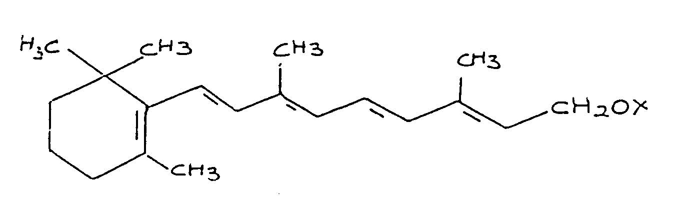
où X représente H ou -COR, où R représente un groupe choisi parmi des groupes alkyle ou alkényle ramifiés ou non ramifiés présentant une moyenne de 1 à 20 atome(s) de carbone,
dans l'objectif de favoriser la réparation d'une peau photo-endommagée et/ou de réduire ou de prévenir les effets nuisibles du rayonnement U.V. sur la peau,
dans laquelle la quantité d'agent d'éclaircissement de la peau et la quantité de rétinol ou de son dérivé sont exprimées respectivement à la composition totale et dans laquelle la composition est exempte de dérivé de cholécalciférol.

3. Utilisation selon la revendication 1 ou 2, dans laquelle la quantité de rétinol ou d'un de ses dérivés présentant la structure (1) est comprise entre 0,01 et 5 % en masse de la composition.

4. Utilisation selon l'une quelconque des revendications précédentes, dans laquelle la quantité d'agent d'éclaircissement de la peau est comprise entre 0,1 et 10 % en masse de la composition.

5. Utilisation selon l'une quelconque des revendications précédentes, dans laquelle l'agent d'éclaircissement de la peau est de l'hydroquinone ou un de ses dérivés.

6. Utilisation selon l'une quelconque des revendications précédentes, dans laquelle l'agent d'éclaircissement de la peau est de l'hydroquinone.

7. Utilisation selon l'une quelconque des revendications précédentes, dans laquelle la composition comprend du transrétinol.
